# EUROPÄISCHE PATENTSCHRIFT

(11) **EP 2 809 756 B1**
(45) Veröffentlichungstag und Bekanntmachung des Hinweises auf die Patenterteilung: **05.04.2017**
(21) Anmeldenummer: 13700520.3
(22) Anmeldetag: 09.01.2013
(51) Int. Cl.: C11B 9/00, A61K 8/41, A61L 9/01, A61Q 13/00, C11D 3/50, A61Q 19/00, A61K 8/58

(54) **KOMBINATION AUS AMINOALKOHOL, DUFTSTOFF UND KIESELSÄUREESTER UND IHRE VERWENDUNG ALS DUFTSTOFFVORLÄUFER**
COMBINATION OF AN AMINO ALCOHOL, A FRAGRANCE AND A SILICIC ACID ESTER, AND THE USE OF SAME AS A PRO-FRAGRANCE
COMBINAISON D'AMINOALCOOL, DE PARFUM ET D'ESTER D'ACIDE SILICIQUE ET UTILISATION DE LADITE COMBINAISON COMME PRÉCURSEUR DE PARFUM

(30) Priorität: 01.02.2012 DE 102012201422
(43) Veröffentlichungstag der Anmeldung: 10.12.2014
(73) Patentinhaber: Henkel AG & Co. KGaA, 40589 Düsseldorf (DE)
(72) Erfinder: BAUER, Andreas, 41564 Kaarst (DE); HUCHEL, Ursula, 50733 Köln (DE); GERIGK, Andreas, 41812 Erkelenz (DE); WEYHE, Marc, 47802 Krefeld (DE); GERKE, Thomas, 40627 Düsseldorf (DE); SMYREK, Hubert, 47804 Krefeld (DE)
(86) Internationale Anmeldenummer: PCT/EP2013/050245
(87) Internationale Veröffentlichungsnummer: WO 2013/113533

(56) Entgegenhaltungen:
- WO-A1-2008/134212
- WO-A1-2010/142479
- WO-A1-2010/142481
- WO-A1-2011/101182
- WO-A2-2006/119283
- DE-A1- 10 012 949
- DE-A1-102007 012 909

## Beschreibung

Die Erfindung betrifft eine Kombination aus einer Aminoalkohol-Verbindung, einem Duftstoffaldehyd und/oder einem Duftstoffketon und einem Kieselsäureester. Die Erfindung betrifft auch ihre Verwendung als Duftstoffvorläufer und diese enthaltende Wasch- und Reinigungsmittel, Weichspüler und Kosmetika, ein Verfahren zur Verlängerung des Duftempfindens von derartigen Mitteln sowie ein Verfahren zum Abbau von Schlechtgerüchen.

Neben dem direkten Zusatz von Duftstoffen zu Wasch- und Reinigungsmitteln, Weichspülern und Kosmetika wurde auch der Zusatz so genannter Duftstoffvorläufer vorgeschlagen. Duftstoffvorläufer stellen in Analogie zu den Pro-Drugs ein chemisches Derivat eines Duftstoffs dar, das beispielsweise die Flüchtigkeit des Duftstoffs vermindert und unter Umgebungsbedingungen eine zeitlich verzögerte Freisetzung des Duftstoffs erlaubt. Durch eine Derivatisierung von Duftstoffen wie Duftstoffaldehyden, Duftstoffketonen oder Duftstoffalkoholen kann der Dampfdruck dieser Verbindungen erniedrigt werden. Da die Derivatisierungsreaktion reversibel ist, kann der chemisch gebundene Duftstoffaldehyd, das Duftstoffketon oder den Duftstoffalkohol unter bestimmten Bedingungen, beispielsweise Umgebungsbedingungen, an der Bindungsstelle gespalten werden. Dadurch wird der Riechstoff oder Duftstoff wieder freigesetzt, was zu einem verlängerten Dufteindruck führen kann.

In der WO 2007/087977 A1 sind Duftstoffvorläufer beschrieben, die einen Duftstoffaldehyd und/oder ein Duftstoffketon in Form eines bicyclischen Oxazolidins gebunden enthalten.

Aus der DE 19841147 A1 sind Duftstoffvorläufer in Form von Kieselsäureestern bekannt, die Duftstoffalkohole freisetzen.

Es besteht allerdings weiterhin Bedarf Duftstoffvorläufer bereit zu stellen, die einen verlängerten Dufteindruck bei Duftstoffaldehyden, Duftstoffketonen und Duftstoffalkoholen erlauben.

Entsprechend war eine Aufgabe der vorliegenden Anmeldung, Möglichkeiten, die einen verlängerten Dufteindruck bei Duftstoffaldehyden, Duftstoffketonen und Duftstoffalkoholen erlauben, bereitzustellen.

Eine weitere Aufgabe lag darin, eine Möglichkeit zum Abbau von Schlechtgerüchen bereit zu stellen.

Überraschenderweise wurde nun gefunden, dass die Kombination aus
(a) einer Aminoalkohol-Verbindung der allgemeinen Formel (I) 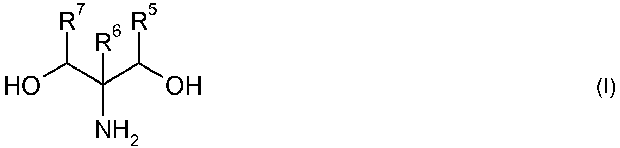 wobei R⁵, R⁶, R⁷ unabhängig voneinander für H oder einen Kohlenwasserstoff-Rest stehen, der acyclisch oder cyclisch, substituiert oder unsubstituiert, verzweigt oder unverzweigt sowie gesättigt oder ungesättigt sein kann,
   oder Gemische dieser Verbindungen,
(b) einem Duftstoffaldehyd und/oder Duftstoffketon und
(c) einem Kieselsäureester der allgemeinen Formel (II): 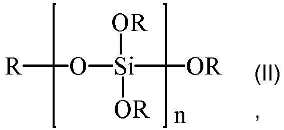 wobei alle R unabhängig voneinander ausgewählt sind aus der Gruppe, die H, die geradkettigen oder verzweigten, gesättigten oder ungesättigten, substituierten oder unsubstituierten C₁₋₆-Kohlenwasserstoffreste und die Duftstoffalkoholreste enthält, und n Werte aus dem Bereich 2 bis 100 annimmt, zu einem verlängerten Dufteindruck bei Duftstoffaldehyden, Duftstoffketonen sowie Duftstoffalkoholen und gleichzeitig zu einem verbesserten Abbau von Schlechtgerüchen führt.

Wobei mindestens 10 mol-%, vorzugsweise mindestens 20 mol-% und insbesondere bevorzugt sogar mehr als 40 mol-% der Reste R der allgemeinen Formel (II) ausgewählt sind aus der Gruppe bestehend aus den Resten der Duftstoffalkohole 10-Undecen-1-ol, 2,6-Dimethylheptan-2-ol, 2-Methylbutanol, 2-Methylpentanol, 2-Phenoxyethanol, 2-Phenylpropanol, 2-tert-Butylcyclohexanol, 3,5,5-Trimethylcyclohexanol, 3-Hexanol, 3-Methyl-5-phenylpentanol, 3- Octanol, 3-Phenylpropanol, 4-Heptenol, 4-Isopropylcyclohexanol, 4-tert-Butylcyclohexanol, 6,8-Dimethyl-2-nonanol, 6-Nonen-1 -ol, 9-Decen-1-ol, alpha-Methylbenzylalkohol, alphaTerpineol, Amylsalicylat, Benzylalkohol, Benzylsalicylat, beta-Terpineol, Butylsalicylat, Citronellol, Cyclohexylsalicylat, Decanol, Dihydromyrcenol, Dimethylbenzylcarbinol, Dimethylheptanol, Dimethyloctanol, Ethylsalicylat, Ethylvanillin, Eugenol, Farnesol, Geraniol, Heptanol, Hexylsalicylat, Isoborneol, Isoeugenol, Isopulegol, Linalool, Menthol, Myrtenol, n-Hexanol, Nerol, Nonanol, Octanol, p-Menthan-7-ol, Phenylethylalkohol, Phenol, Phenylsalicylat, Tetrahydrogeraniol, Tetrahydrolinalool, Thymol, trans-2-cis-6-Nonadienol, trans-2-Nonen-1- ol, trans-2-Octenol, Undecanol, Vanillin, Zimtalkohol und Mischungen daraus.

Es ist bevorzugt, dass der Duftstoffaldehyd und/oder das Duftstoffketon zumindest teilweise unter Ringschluss mit der Aminoalkohol-Verbindung der allgemeinen Formel (I) zu einer 1-Aza-3,7-dioxabicyclo[3.3.0]octan-Verbindung der allgemeinen Formel (III) 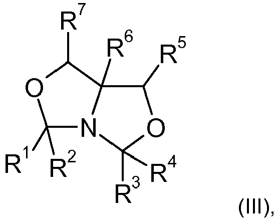 wobei R¹, R², R³, R⁴ unabhängig voneinander für Reste stehen, die in einer Verbindung der allgemeinen Formel R¹-C(=O)-R² oder R³-C(=O)-R⁴ einen Duftstoffaldehyd mit mindestens 6 Kohlenstoffatomen oder ein Duftstoffketon mit mindestens 6 Kohlenstoffatomen ergeben, reagiert.

Ohne an diese Theorie gebunden sein zu wollen, wird vermutet, dass die Aminoalkohol-Verbindung mit dem Duftstoffaldehyd bzw. dem Duftstoffketon unter Ausbildung bicyclischer Oxazolidin-Derivate reagiert. Bicyclische Oxazolidin-Derivate von Duftstoffaldehyden und Duftstoffketonen führen zu einer Verminderung des Dampfdrucks der Duftstoffaldehyde und Duftstoffketone und einer Verlängerung des Dufteindrucks. Zudem ist die Ablagerung der bicyclischen Verbindungen auf festen Oberflächen wie Textilien, Haut oder harten Oberflächen verbessert.

Die Verbindungen der allgemeinen Formel (I) leiten sich von 2-Amino-1,3-propandiol ab. Durch Bildung der bicyclischen Verbindungen ist es möglich, einen hohen Beladungsgrad der 2-Amino-1,3-propandiole zu erreichen, so dass der Einsatz geringer Mengen der 2-Amino-1,3-propandiole erforderlich ist. Damit kann eine Verlängerung des Dufteindrucks bereits mit geringeren Mengen an 2-Amino-1,3-propandiolen erreicht werden, was unter anderem zu Kostenvorteilen führen kann und auch den Eintrag größerer Mengen an Aminoalkohol-Verbindung in Mittel, beispielsweise Waschoder Reinigungsmittel, Weichspüler oder Kosmetika, vermeidet.

In insbesondere bevorzugten Verbindungen der allgemeinen Formel (III) bedeuten R², R⁴, R⁵, R⁶, R⁷ Wasserstoff und R¹ und R³ jeweils einen C₅₋₂₄-Kohlenwasserstoffrest.

Es ist insbesondere bevorzugt, dass die Duftstoffaldehyde und die Duftstoffketone ausgewählt sind aus den Jasmonen, lononen, Damasconen und Damascenonen, Menthon, Carvon, Iso-E-Super, Methylheptenon, Melonal, Cymal, Ethylvanillin, Helional, Hydroxycitronellal, Koavon, Methylnonylacetaldehyd, Phenylacetaldehyd, Undecylenaldehyd, 3-Dodecen-I-al, alpha-n-Amylzimtaldehyd, Benzaldehyd, 3-(4-tert-Butylphenyl)-propanal, 2-Methyl-3-(para-methoxyphenylpropanal), 2-Methyl-4-(2,6,6-trimethyl-2(1)-cyclohexen-1-yl)butanal, 3-Phenyl-2-propenal, cis-/trans-3,7-Dimethyl-2,6-octadien-I-al, 3,7-Dimethyl-6-octen-I-al, [(3,7-Dimethyl-6-octenyl)oxy]acetaldehyd, 4-Isopropyl-benzyaldehyd, 2,4-Dimethyl-3-cyclohexen-1-carboxyaldehyd, 2-Methyl-3-(isopropylphenyl)propanal, Decylaldehyd, 2,6-Dimethyl-5-heptenal, alpha-n-Hexylzimtaldehyd, 7-Hydroxy-3,7-dimethyl octanal, Undecenal, 2,4,6-Trimethyl-3-cyclohexene-I-carboxaldehyd, 1-Dodecanal, 2,4-Dimethyl-cyclohexene-3-carboxaldehyd, 4-(4-Hydroxy-4-methyl pentyl)-3-cylohexene-I-carboxaldehyd, 2-Methyl undecanal, 2-Methyl decanal, 1-nonanal, 1-Octanal, 2,6,10-Trimethyl-5,9-undecadienal, 2-Methyl-3-(4-tertbutyl)propanal, Dihydrozimtaldehyd, 3,7-Dimethyloctan-1-al, 1-Undecanal,10-Undecen-1-al, 4-Hydroxy-3-methoxybenzaldehyd, trans-4-decenal, 2,6-Nonadienal, para-Tolylacetaldehyd, 3,7-Dimethyl-2-methylene-6-octenal, 2-Methyloctanal, alpha-Methyl-4-(I-methyl-ethyl)benzeneacetaldehyd, 2-methyl-3-phenyl-2-propen-1-al, 3,5,5-Trimethylhexanal, 3-Propyl-bicyclo[2.2.1]hept-5-ene-2-carbaldehyd, 9-Decenal, 3-Methyl-5-phenyl-1-pentanal, Methylnonylacetaldehyd, Citral, 1-Decanal, Florhydral, 2,4-Dimethyl-3-cyclohexen-1-carboxaldehyd, Heliotropin und Mischungen daraus.

Diese Duftstoffaldehyde und die Duftstoffketone eigenen sich besonders gut, um Mitteln einen angenehmen Duft und/oder eine Frischeempfinden zu vermitteln.

Es kann auch bevorzugt sein, dass der Duftstoffaldehyd und/oder das Duftstoffketon zumindest teilweise unter Ringschluss mit der Aminoalkohol-Verbindung der allgemeinen Formel (I) zu einer Verbindung der allgemeinen Formel (IV) 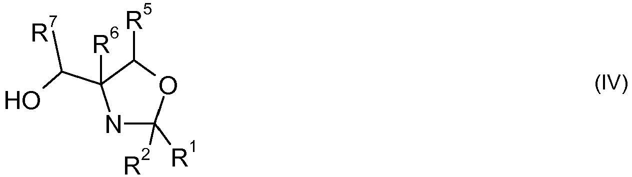 wobei R¹ und R² unabhängig voneinander für Reste stehen, die in einer Verbindung der allgemeinen Formel R¹-C(=O)-R² einen Duftstoffaldehyd mit mindestens 6 Kohlenstoffatomen oder ein Duftstoffketon mit mindestens 6 Kohlenstoffatomen ergeben, reagiert.

Es ist erfindungsgemäß auch möglich, dass sich Gemische von einfach und zweifach geschlossenen Verbindungen auf Grundlage der 2-Amino-1,3-propandiol-Derivate bilden.

In einer bevorzugten Ausführungsform der Erfindung ist ein Teil der Reste R der allgemeinen Formel (II), vorzugsweise mindestens 5 mol-% der Reste R ausgewählt aus der Gruppe Methyl, Ethyl, n-Propyl, iso-Propyl, n-Butyl, iso-Butyl und tert-Butyl.

Oligokieselsäureester niederer Alkohole sind kommerziell erhältlich, wobei üblicherweise Methanol, Ethanol, n-Propanol, iso-Propanol, n-Butanol, iso-Butanol und tert-Butanol zur Veresterung eingesetzt wurden. Die Herstellung nicht vollständig mit Duftstoffalkoholen umgeesterter Oligokieselsäureester führt zu Kieselsäureester-Mischungen in denen ein Teil der Reste R ausgewählt ist aus der Gruppe Methyl, Ethyl, n-Propyl, iso-Propyl, n-Butyl, iso-Butyl und tert-Butyl. Solche Verbindungen sind im Rahmen der vorliegenden Erfindung bevorzugt.

Werden nicht-vollständig umgeesterte Oligokieselsäureester hergestellt, sind die übrigen Reste R vorzugsweise aus der Gruppe der Duftstoffalkoholreste ausgewählt.

Mindestens 10 mol-%, vorzugsweise mindestens 20 mol-% und insbesondere bevorzugt sogar mehr als 40 mol-% der Reste R der allgemeinen Formel (II) sind ausgewählt aus der Gruppe bestehend aus den Resten der Duftstoffalkohole 10-Undecen-1-ol, 2,6-Dimethylheptan-2-ol, 2-Methyl-butanol, 2-Methylpentanol, 2-Phenoxyethanol, 2-Phenyl-propanol, 2-tert-Butylcyclohexanol, 3,5,5-Trimethylcyclohexanol, 3-Hexanol, 3-Methyl-5-phenylpentanol, 3-Octanol, 3-Phenylpropanol, 4-Heptenol, 4-Isopropylcyclohexanol, 4-tert-Butylcyclohexanol, 6,8-Dimethyl-2-nonanol, 6-Nonen-1-ol, 9-Decen-1-ol, alpha-Methylbenzylalkohol, alpha-Terpineol, Amylsalicylat, Benzylalkohol, Benzylsalicylat, beta-Terpineol, Butylsalicylat, Citronellol, Cyclohexylsalicylat, Decanol, Dihydromyrcenol, Dimethylbenzylcarbinol, Dimethylheptanol, Dimethyloctanol, Ethylsalicylat, Ethylvanillin, Eugenol, Farnesol, Geraniol, Heptanol, Hexylsalicylat, Isoborneol, Isoeugenol, Isopulegol, Linalool, Menthol, Myrtenol, n-Hexanol, Nerol, Nonanol, Octanol, p-Menthan-7-ol, Phenylethylalkohol, Phenol, Phenylsalicylat, Tetrahydrogeraniol, Tetrahydrolinalool, Thymol, trans-2-cis-6-Nonadienol trans-2-Octenol, Undecanol, Vanillin, Zimtalkohol und Mischungen daraus.

In einer weiteren, bevorzugten Ausführungsform nimmt n Werte aus dem Bereich von 2 bis 50, vorzugsweise aus dem Bereich von 2 bis 20 und insbesondere aus dem Bereich von 3 bis 10 unter besonderer Bevorzugung der Werte 4, 5, 6, 7 und 8, an.

Da die Ausgangsverbindungen für die Herstellung der Kieselsäureester aus ökonomischen Gründen vorzugsweise keine Reinstoffe sind, sondern technische Gemische von Oligokieselsäureestern niederer Alkohole mit unterschiedlichen Oligomerisierungsgraden, findet sich eine Verteilung der Oligomerisierungsgrade auch in den erfindungsgemäßen Estern wieder, die dem Ausgangsmaterial entsprechen kann oder durch die Reaktionsbedingungen modifiziert ist.

Die Erfindung betrifft auch die Verwendung der Kombination aus (a) einer Aminoalkohol-Verbindung der allgemeinen Formel (I), (b) einem Duftstoffaldehyd und/oder Duftstoffketon und (c) einem Kieselsäureester der allgemeinen Formel (II) als Duftstoffvorläufer. Dabei kann es insbesondere bevorzugt sein, dass die Kombination zusammen mit anderen Duftstoffen eingesetzt wird.

Die Erfindung betrifft ebenso Wasch- oder Reinigungsmittel, Weichspüler oder Kosmetika, die die Kombination aus (a) einer Aminoalkohol-Verbindung der allgemeinen Formel (I), (b) einem Duftstoffaldehyd und/oder Duftstoffketon und (c) einem Kieselsäureester der allgemeinen Formel (II) enthalten.

Die Erfindung betrifft ferner ein Verfahren zur Verlängerung des Duftempfindens von Wasch- und Reinigungsmitteln, Weichspülern und Kosmetika oder mit diesen behandelten festen Oberflächen, wobei man den Wasch- oder Reinigungsmitteln, Weichspülern oder Kosmetika die Kombination aus (a) einer Aminoalkohol-Verbindung der allgemeinen Formel (I), (b) einem Duftstoffaldehyd und/oder Duftstoffketon und (c) einem Kieselsäureester der allgemeinen Formel (II) zusetzt.

Ferner betrifft die Erfindung ein Verfahren zum Abbau von Schlechtgerüchen durch Einsatz einer Kombination aus (a) einer Aminoalkohol-Verbindung der allgemeinen Formel (I), (b) einem Duftstoffaldehyd und/oder Duftstoffketon und (c) einem Kieselsäureester der allgemeinen Formel (II).

Der Begriff Schlechtgeruch ist dem Fachmann allgemein bekannt. Unter Schlechtgeruch im Sinne dieser Anmeldung werden alle Gerüche einbezogen, die in einer Gruppe von zehn Personen von mindestens 7 dieser Personen als unangenehm/schlecht eingestuft werden. Beispiele solcher Schlechtgerüche sind Schweißgeruch, Fäkalgeruch, Modergeruch, Bakteriell erzeugter Schlechtgeruch, Fischgeruch, der Geruch von C₁- bis C₁₅-Fettsäuren oder der Geruch von stundenlang stehender, nasser Wäsche.

Der Abbau von Schlechtgerüchen umfasst insbesondere die Bekämpfung und/oder Überdeckung von Schlechtgerüchen. Die Kombination aus (a) ausgewählter Aminoalkohol-Verbindung, (b) Duftstoffaldehyd und/oder ein Duftstoffketon und (c) ausgewähltem Kieselsäureester weist synergistische Eigenschaften beim Abbau von Schlechtgerüchen, insbesondere über einen Zeitraum von mehreren Tagen auf.

Im Folgenden soll die Erfindung, unter anderem anhand von Beispielen, eingehender erläutert werden.

Als essentielle Bestandteile enthält die Kombination (a) eine ausgewählte Aminoalkohol-Verbindung, (b) einen Duftstoffaldehyd und/oder ein Duftstoffketon und (c) einen ausgewählten Kieselsäureester.

Die Kombination enthält eine Aminoalkohol-Verbindung der allgemeinen Formel (I)

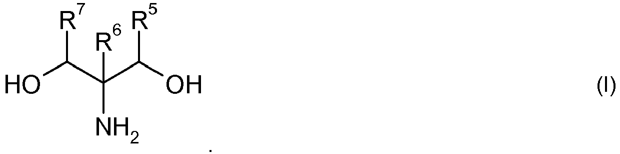

Bei den Aminoalkohol-Verbindungen handelt es sich um 2-Amino-1,3-propandiole. R⁶ kann Wasserstoff oder Alkyl bedeuten, das durch ein oder zwei Hydroxylgruppen und/oder eine Aminogruppe substituiert sein kann, wobei auch bis zu 8 nicht benachbarte -CH₂-Gruppen durch -O- ersetzt sein können. Alkylreste sind dabei bevorzugt C₁₋₂₄-Alkylreste, besonders bevorzugt C₁-₁₆-Alkylreste, insbesondere C₁₋₁₂-Alkylreste, speziell C₁₋₆-Alkylreste, beispielhaft C₁₋₃-Alkylreste. Alkylreste können dabei linear, verzweigt oder cyclisch sein. Vorzugsweise handelt es sich um lineare Alkylreste. Es kann sich um Mono- oder Dihydroxyalkylreste handeln, die anstelle der Hydroxylgruppen oder zusätzlich auch eine Aminogruppe aufweisen können. Die Alkylreste können ferner substituiert oder unsubstituiert sein. Sofern die Alkylreste durch -O- unterbrochen sind, handelt es sich vorzugsweise um Strukturelemente der Formel -CH₂-CH₂-O- oder -CH₂-CH(CH₃)-O-. Derartige Verbindungen sind in einfacher Weise durch Alkoxylierung der entsprechenden Hydroxyverbindungen zugänglich.

Besonders bevorzugte Reste R⁶ sind Methyl-, Ethyl-, und Hydroxymethylreste.

R⁵ und R⁷ sind Wasserstoff oder ein C₁₋₆-Alkylrest, vorzugsweise C₁₋₃-Alkylrest. Besonders bevorzugt sind R⁵ und R⁷ Wasserstoff oder ein Methyl- oder Ethylrest, insbesondere Wasserstoff.

Bevorzugte Aminoalkohol-Verbindungen sind 2-Amino-1,3-propandiol, 2-Amino-2-hydroxymethyl-1,3-propandiol, 2-Amino-2-methyl-1,3-propandiol, 2-Amino-2-ethyl-1,3-propandiol und Mischungen daraus.

Die Kombination enthält ferner einen Duftstoffaldehyd und/oder ein Duftstoffketon. Dies bedeutet, dass die Kombination nur einen Typ Duftstoffaldehyd oder nur einen Typ Duftstoffketon enthalten kann. Davon umfasst ist aber auch, dass die Kombination einen Typ Duftstoffaldehyd und einen Typ Duftstoffketon enthalten kann. Ferner ist umfasst, dass die Kombinationen mehrere Typen Duftstoffaldehyd oder mehrere Typen Duftstoffketon enthalten können. Ebenso ist es möglich, dass die Kombination mehrere Typen Duftstoffaldehyd und einen Typ Duftstoffketon oder mehrere Typen Duftstoffaldehyd und mehrere Typen Duftstoffketon enthält. In einer weiteren Ausführungsform enthält die Kombination mehrere Typen Duftstoffketon und einen Typ Duftstoffaldehyd oder mehrere Typen Duftstoffketon und mehrere Typen Duftstoffaldehyde.

Die Duftstoffketone können alle Ketone umfassen, die einen erwünschten Duft oder ein Frischeempfinden verleihen können. Es können auch Gemische unterschiedlicher Ketone eingesetzt werden. Beispielsweise kann das Keton ausgewählt sein aus der Gruppe, bestehend aus Buccoxim, Iso-jasmon, Methyl-beta-naphthylketon, Moschus indanon, Tonalid/Moschus plus, Alpha-Damascone, Beta-Damascone, Delta-Damascone, Iso-Damascone, Damascenone, Damarose, Methyldihydrojasmonat, Menthon, Carvon, Campher, Fenchon, Alpha-Ionen, Beta-Ionon, Dihydro-Beta-Ionon, Gamma-Methyl so genanntes Ionon, Fleuramon, Dihydrojasmon, Cis-Jasmon, Iso-E-Super, Methylcedrenylketon oder Methylcedrylon, Acetophenon, Methylacetophenon, Para-Methoxyacetophenon, Methyl-beta-naphtylketon, Benzylaceton, Benzophenon, Para-Hydroxyphenylbutanon, Celery Keton oder Livescon, 6-Isopropyldecahydro-2-naphton, Dimethyloctenon, Freskomenth, 4-(1-Ethoxyvinyl)-3,3,5,5,-tetramethyl-cyclohexanon, Methylheptenon, 2-(2-(4-Methyl-3-cyclohexen-1-yl)propyl)-cyclopentanon, 1-(p-Menthen-6(2)-yl)-1-propanon, 4-(4-Hydroxy-3-methoxyphenyl)-2-butanon, 2-Acetyl-3,3-dimethyl-norbornan, 6,7-Dihydro-1,1,2,3,3-pentamethyl-4(5H)-indanon, 4-Damascol, Dulcinyl oder Cassion, Gelson, Hexalon, Isocyclemon E, Methylcyclocitron, Methyl-Lavendel-keton, Orivon, Para-tert-butyl-cyclohexanon, Verdon, Delphon, Muscon, Neobutenon, Plicaton, Velouton, 2,4,4,7-Tetramethyl-oct-6-en-3-on, Tetrameran, Hedion und Gemischen davon. Bevorzugt können die Ketone ausgewählt sein aus Alpha-Damascone, Delta-Damascone, Iso-Damascone, Carvon, Gamma-Methyl-Ionen, Iso-E-Super, 2,4,4,7-Tetramethyl-oct-6-en-3-on, Benzylaceton, Beta Damascon, Damascenone, Methyldihydrojasmonat, Methylcedrylon, Hedion und Gemischen davon.

Geeignete Duftstoffaldehyde können beliebige Aldehyde sein, die entsprechend der Duftstoffketone einen gewünschten Duft oder eine Frischeempfinden vermitteln. Es kann sich wiederum um einzelne Aldehyde oder Aldehydgemische handeln. Geeignete Aldehyde sind beispielsweise Melonal, Triplal, Ligustral, Adoxal, Anisaldehyd, Cymal, Ethylvanillin, Florhydral, Helional, Heliotropin, Hydroxycitronellal, Koavon, Laurinaldehyd, Lyral, Methyl-nonyl-acetaldehyd, p,t-Bucinal, Phenylacetaldehyd, Undecylenaldehyd, Vanillin,2,6,10-Trimethyl-9-undecenal, 3-Dodecen-I-al, alpha-n-Amylzimtaldehyd, 4-Methoxybenzaldehyd, Benzaldehyd, 3-(4-tert-Butylphenyl)-propanal, 2-Methyl-3-(para-methoxyphenylpropanal), 2-Methyl-4-(2,6,6-trimethyl-2(1)-cyclohexen-1-yl)butanal, 3-Phenyl-2-propenal, cis-/trans-3,7-Dimethyl-2,6-octadien-I-al, 3,7-Dimethyl-6-octen-I-al, [(3,7-Dimethyl-6-octenyl)oxy]acetaldehyd, 4-Isopropylbenzyaldehyd, 1,2,3,4,5,6,7,8-octahydro-8,8-Dimethyl-2-naphthaldehyd, 2,4-Dimethyl-3-cyclohexen-1-carboxyaldehyd, 2-Methyl-3-(isopropylphenyl)propanal,Decyl aldehyd, 2,6-Dimethyl-5-heptenal, 4-(tricyclo[5.2. 1.0 (2,6)]-decylidene-8)-butanal, Octahydro-4,7-methano-IH-indenecarboxaldehyd, 3-Ethoxy-4-hydroxybenzaldehyd, para-Ethyl-alpha, alpha-Dimethylhydrozimtaldehyd, alpha-Methyl-3,4-(methylenedioxy)-hydrozimtaldehyd, 3,4-Methylenedioxybenzaldehyd, alpha-n-Hexylzimtaldehyd, m-Cymene-7-carboxaldehyd, alpha-Methylphenylacetaldehyd, 7-Hydroxy-3,7-dimethyloctanal, Undecenal, 2,4,6-Trimethyl-3-cyclohexene-I-carboxaldehyd, 4-(3)(4-Methyl-3-pentenyl)-3-cyclohexen-carboxaldehyd, 1-Dodecanal, 2,4-Dimethyl cyclohexene-3-carboxaldehyd, 4-(4-Hydroxy-4-methyl pentyl)-3-cylohexene-I-carboxaldehyd, 7-Methoxy-3,7-dimethyloctan-1-al, 2-Methyl undecanal, 2-Methyl decanal,1-nonanal, 1-Octanal, 2,6,10-Trimethyl-5,9-undecadienal, 2-Methyl-3-(4-tertbutyl)propanal, Dihydrozimtaldehyd,1-methyl-4-(4-methyl-3-pentenyl)-3-cyclohexene-1-carboxaldehyd, 5 oder 6 Methoxyhexahydro-4,7-methanoindan-1 oder 2-carboxyaldehyd, 3,7-Dimethyloctan-1-al, 1-Undecanal,10-Undecen-1-al, 4-Hydroxy-3-methoxybenzaldehyd, 1-Methyl-3-(4-methylpentyl)-3-cyclohexenecarboxyaldehyd, 7-Hydroxy-3,7-dimethyl-octanal, trans-4-decenal, 2,6-Nonadienal, para-Tolylacetaldehyd, 4-Methylphenylacetaldehyd, 2-Methyl-4-(2,6,6-trimethyl-1-cyclohexen-1-yl)-2-butenal, ortho-Methoxyzimtaldehyd, 3,5,6-Trimethyl-3-cyclohexenecarboxaldehyd, 3,7-Dimethyl-2-methylene-6-octenal, Phenoxyacetaldehyd, 5,9-Dimethyl-4,8-decadienal, Peony aldehyd (6,10-dimethyl-3-oxa-5,9-undecadien-1-al), Hexahydro-4,7-methanoindan-1-carboxaldehyd, 2-Methyl octanal, alpha-Methyl-4-(I-methylethyl) benzeneacetaldehyd, 6,6-Dimethyl-2-norpinene-2-propionaldehyd, para-Methylphenoxyacetaldehyd, 2-methyl-3-phenyl-2-propen-1-al, 3,5,5-Trimethylhexanal, Hexahydro-8,8-dimethyl-2-naphthaldehyd, 3-Propyl-bicyclo [2.2.1]-hept-5-ene-2-carbaldehyd, 9-Decenal, 3-Methyl-5-phenyl-1-pentanal, Methylnonylacetaldehyd, 1-p-Menthene-q-carboxaldehyd, Citral oder Gemische davon, Lilial citral, 1-Decanal, Florhydral, 2,4-Dimethyl-3-cyclohexen-1-carboxaldehyd, bevorzugte Aldehyde können ausgewählt sein aus cis/trans-3,7-Dimethyl-2,6-octadien-I-al, Heliotropin, 2,4,6-Trimethyl-3-cyclohexene-I-carboxaldehyd, 2,6-Nonadienal, alpha-n-Amylzimtaldehyd, alpha-n-Hexylzimtaldehyd, p-tert-Bucinal, Lyral, Cymal, Methylnonylacetaldehyd, trans-2-Nonenal, Lilial, trans-2-Nonenal und Gemische davon.

Wie vorstehend beispielhaft ausgeführt, können die Duftstoffaldehyde und/oder Duftstoffketone eine aliphatische, cycloaliphatische, aromatische, ethylenisch ungesättigte Struktur oder eine Kombination dieser Strukturen aufweisen. Es können ferner weitere Heteroatome oder polycyclische Strukturen vorliegen. Die Strukturen können geeignete Substituenten wie Hydroxyl- oder Aminogruppen aufweisen.

Die aufgeführten Duftstoffaldehyde und/oder Duftstoffketone weisen zudem besonders gute Ergebnisse bei der Bekämpfung und Überdeckung von Schlechtgerüchen auf.

Als dritten essentiellen Bestandteil enthält die Kombination einen Kieselsäureester der allgemeinen Formel (II) 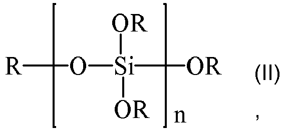 wobei alle R unabhängig voneinander ausgewählt sind aus der Gruppe, die H, die geradkettigen oder verzweigten, gesättigten oder ungesättigten, substituierten oder unsubstituierten C₁₋₆-Kohlenwasserstoffreste und die Duftstoffalkoholreste enthält, und n Werte aus dem Bereich 2 bis 100 annimmt.

Die Herstellung der genannten Verbindungen gelingt durch einfache Umesterung von Oligokieselsäureestern niederer Alkohole mit Duftstoffalkoholen, wobei sowohl einzelne Duftstoffalkohole als auch Duftstoffalkoholgemische eingesetzt werden können. Je nach Reaktionszeit und -bedingungen werden die niederen Alkohole abgespalten und die Duftstoff- bzw. Biozidalkohole gebunden, wobei die Alkohole entlang von Si-O-Si-Ketten oder -Ringen leichter ausgetauscht werden als die terminalen Alkohole. Üblicherweise werden als Edukte die handelsüblichen Kieselsäureester eingesetzt. Hier ist insbesondere der Ethanol-Ester zu nennen. Die Umesterung kann dabei ausschließlich durch Temperaturerhöhung und Abdestillation der leichtflüchtigen Nebenprodukte gesteuert werden. bevorzugt ist es jedoch, wenn zur Umesterung Katalysatoren eingesetzt werden. Es handelt sich dabei üblicherweise um Lewis-Säuren, vorzugsweise um Aluminiumtetraisopropylat, Titantetraisopropylat, Siliciumtetrachlorid oder basische Katalysatoren oder auch um Zubereitungen wie beispielsweise aus Aluminiumoxid mit Kaliumfluorid. Die so gebildeten oligomeren Kieselsäureester weisen dann zumindest teilweise Duftstoffalkoholreste auf. Üblicherweise enthalten die resultierenden Ester jedoch auch noch Reste niederer Alkohole. Soweit bei der Herstellung der Kieselsäureester geringe Mengen Wasser oder anderer Wasserstoff-acider Verbindungen anwesend sind, findet auch Austausch von Alkohol-Resten gegen OH-Gruppen statt. Dementsprechend enthalten die erfindungsgemäßen Kieselsäureester-Mischungen üblicherweise als Rest R teilweise auch Wasserstoff.

Oligokieselsäureester niederer Alkohole sind kommerziell erhältlich, wobei üblicherweise Methanol, Ethanol, n-Propanol, iso-Propanol, n-Butanol, iso-Butanol und tert-Butanol zur Veresterung eingesetzt wurden. Die Darstellung nicht vollständig mit Duftstoffalkoholen umgeesterter Oligokieselsäureester führt zu Kieselsäureester-Mischungen in denen ein Teil der Reste R ausgewählt ist aus der Gruppe Methyl, Ethyl, n-Propyl, iso-Propyl, n-Butyl, iso-Butyl und tert-Butyl.

Werden nicht-vollständig umgeesterte Oligokieselsäureester hergestellt, sind die übrigen Reste R vorzugsweise aus der Gruppe der Duftstoffalkoholreste ausgewählt.

Unter dem Begriff "Duftstoffalkohole" werden im Rahmen der vorliegenden Erfindung Duftstoffe verstanden, die über freie Hydroxylgruppen verfügen, welche veresterbar sind, unabhängig davon, wie das Molekül weiter aufgebaut ist. So lassen sich auch Salicylsäureester als Duftstoffalkohole einsetzen. Es werden Duftstoffalkohole ausgewählt, so dass im Rahmen der vorliegenden Erfindung Kieselsäureester verwendet werden, in denen jedes R und R unabhängig voneinander ausgewählt ist aus der Gruppe der Reste folgender Duftstoffalkohole: 10-Undecen-1-ol, 2,6-Dimethylheptan-2-ol, 2-Methylbutanol, 2-Methylpentanol, 2-Phenoxyethanol, 2-Phenyl-propanol, 2-tert-Butycyclohexanol, 3,5,5-Trimethylcyclohexanol, 3-Hexanol, 3-Methyl-5-phenylpentanol, 3-Octanol, 3-Phenylpropanol, 4-Heptenol, 4-Isopropylcyclo-hexanol, 4-tert-Butycyclohexanol, 6,8-Dimethyl-2-nonanol, 6-Nonen-1-ol, 9-Decen-1-ol, alpha-Methyl-benzylalkohol, alpha-Terpineol, Amylsalicylat, Benzylalkohol, Benzylsalicylat, beta-Terpineol, Butylsalicylat, Citronellol, Cyclohexylsalicylat, Decanol, Dihydromyrcenol, Dimethylbenzylcarbinol, Dimethylheptanol, Dimethyloctanol, Ethylsalicylat, Ethylvanillin, Eugenol, Farnesol, Geraniol, Heptanol, Hexylsalicylat, Isoborneol, Isoeugenol, Isopulegol, Linalool, Menthol, Myrtenol, n-Hexanol, Nerol, Nonanol, Octanol, para-Menthan-7-ol, Phenylethylalkohol, Phenol, Phenylsalicylat, Tetrahydrogeraniol, Tetrahydrolinalool, Thymol, trans-2-cis-6-Nonadienol trans-2-Nonen-1-ol, trans-2-Octenol, Undecanol, Vanillin, Zimtalkohol und Mischungen daraus.

Bevorzugt werden Phenylethylkieselsäureester, Geranylkieselsäureester, Citronellylkieselsäureester, Cynamylkieselsäureester, Hexenylkieselsäureester, Nonadienylkieselsäureester, Octenylkieselsäureester oder Mischungen aus zwei oder mehr dieser Kieselsäureester eingesetzt.

Die Menge an Aminoalkohol der allgemeinen Formel (I) in der Kombination beträgt vorzugsweise 0,5 bis 50 Gew.-%, bezogen auf die Gesamtmenge an Kombination. Die Menge Duftstoffaldehyd und/oder Duftstoffketon in der Kombination beträgt vorzugsweise 0,5 bis 50 Gew.-%, bezogen auf die Gesamtmenge an Kombination. Die Menge an Kieselsäureester der allgemeinen Formel (II) in der Kombination beträgt vorzugsweise 0,5 bis 50 Gew.-%, bezogen auf die Gesamtmenge an Kombination.

Die Kombination aus (a) ausgewählter Aminoalkohol-Verbindung, (b) Duftstoffaldehyd und/oder Duftstoffketon und (c) ausgewähltem Kieselsäureester wird vorzugsweise als Duftstoffvorläufer eingesetzt. Der Begriff "Duftstoffvorläufer" beschreibt dabei Derivate von Duftstoffaldehyden und Duftstoffketonen, die unter Umgebungsbedingungen die ursprünglichen Duftstoffaldehyde und Duftstoffketone freisetzen. Umgebungsbedingungen sind dabei die typischen Umgebungsbedingungen im menschlichen Lebensraum bzw. die auf der menschlichen Haut anzutreffenden Bedingungen. Unter Umgebungsbedingungen zerfallen die Verbindungen der allgemeinen Formel (I) in Umkehr des Herstellungsverfahrens langsam unter Freisetzung der ursprünglichen Duftstoffaldehyde und/oder Duftstoffketone. Die chemisch gebundenen Duftstoffaldehyde und Duftstoffketone werden an der Bindungsstelle gespalten, wodurch die Duftstoffe wieder freigesetzt werden.

Demgemäß ist die Verwendung der beschriebenen Kombination als Duftstoffvorläufer, welche vorzugsweise als Duftstoffe Duftstoffaldehyde und/oder Duftstoffketone freisetzt, besonders bevorzugt.

Die offenbarte Kombination kann alleine, beispielsweise als Parfüm, eingesetzt werden, es ist aber auch möglich, Duftstoffgemische einzusetzen, die lediglich zu einem Teil aus der beschriebenen Kombination bestehen.

In einer bevorzugten Ausführungsform enthält ein Parfüm eine Kombination aus
(a) einer Aminoalkohol-Verbindung der allgemeinen Formel (I) 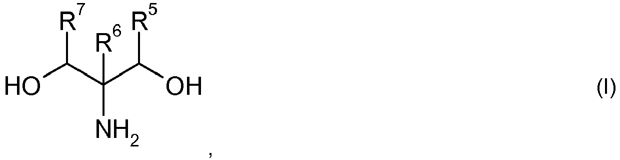 wobei R⁵, R⁶, R⁷ unabhängig voneinander für H oder einen Kohlenwasserstoff-Rest stehen, der acyclisch oder cyclisch, substituiert oder unsubstituiert, verzweigt oder unverzweigt sowie gesättigt oder ungesättigt sein kann,
   oder Gemische dieser Verbindungen,
(b) einem Duftaldehyd oder Duftketon und
(c) einem Kieselsäureester der allgemeinen Formel (II): 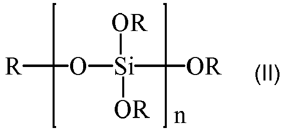 wobei alle R unabhängig voneinander ausgewählt sind aus der Gruppe, die H, die geradkettigen oder verzweigten, gesättigten oder ungesättigten, substituierten oder unsubstituierten C₁₋₆-Kohlenwasserstoffreste und die Duftstoffalkoholreste enthält, und n Werte aus dem Bereich 2 bis 100 annimmt.

Wobei mindestens 10 mol-%, vorzugsweise mindestens 20 mol-% und insbesondere bevorzugt sogar mehr als 40 mol-% der Reste R der allgemeinen Formel (II) ausgewählt sind aus der Gruppe bestehend aus den Resten der Duftstoffalkohole 10-Undecen-1-ol, 2,6-Dimethylheptan-2-ol, 2-Methylbutanol, 2-Methylpentanol, 2-Phenoxyethanol, 2-Phenylpropanol, 2-tert-Butylcyclohexanol, 3,5,5-Trimethylcyclohexanol, 3-Hexanol, 3-Methyl-5-phenylpentanol, 3- Octanol, 3-Phenylpropanol, 4-Heptenol, 4-Isopropylcyclohexanol, 4-tert-Butycyclohexanol, 6,8-Dimethyl-2-nonanol, 6-Nonen-1 -ol, 9-Decen-1-ol, alpha-Methylbenzylalkohol, alphaTerpineol, Amylsalicylat, Benzylalkohol, Benzylsalicylat, beta-Terpineol, Butylsalicylat, Citronellol, Cyclohexylsalicylat, Decanol, Dihydromyrcenol, Dimethylbenzylcarbinol, Dimethylheptanol, Dimethyloctanol, Ethylsalicylat, Ethylvanillin, Eugenol, Farnesol, Geraniol, Heptanol, Hexylsalicylat, Isoborneol, Isoeugenol, Isopulegol, Linalool, Menthol, Myrtenol, n-Hexanol, Nerol, Nonanol, Octanol, p-Menthan-7-ol, Phenylethylalkohol, Phenol, Phenylsalicylat, Tetrahydrogeraniol, Tetrahydrolinalool, Thymol, trans-2-cis-6-Nonadienol, trans-2-Nonen-1- ol, trans-2-Octenol, Undecanol, Vanillin, Zimtalkohol und Mischungen daraus.

So können insbesondere Parfüme eingesetzt werden, die 0,1 bis 50 Gew.-%, vorzugsweise 0,5 bis 40 Gew.-% und insbesondere maximal 30 Gew.-% an der Kombination aus (a) ausgewählter Aminoalkohol-Verbindung, (b) Duftstoffaldehyd und/oder Duftstoffketon und (c) ausgewähltem Kieselsäureester enthalten.

Durch die Aufteilung des Gesamt-Parfümgehaltes eines Mittels, beispielsweise eines Wasch- oder Reinigungsmittels, in Parfüm, welches in Form der beschriebenen Kombination vorliegt und Parfüm, das herkömmlich eingearbeitet wurde, lässt sich eine Vielzahl von Produktcharakteristiken realisieren, die erst durch die beschriebene Kombination aus (a) ausgewählter Aminoalkohol-Verbindung, (b) Duftstoffaldehyd und/oder Duftstoffketon und (c) ausgewähltem Kieselsäureester möglich werden.

Die Duftstoffe, die auf herkömmliche Weise in die Mittel inkorporiert werden können, sind dagegen keinerlei Beschränkungen unterworfen. So können als Parfümöle bzw. Duftstoffe einzelne Duftstoffverbindungen natürlichen oder synthetischen Ursprungs, z.B. vom Typ der Ester, Ether, Aldehyde, Ketone, Alkohole und Kohlenwasserstoffe verwendet werden. Duftstoffverbindungen vom Typ der Ester sind z.B. Benzylacetat, Phenoxyethylisobutyrat, p-tert-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmacyclat. Zu den Ethern zählen beispielsweise Benzylethylether und Ambroxan, zu den Aldehyden z.B. die linearen Alkanale mit 8 bis 18 C-Atomen, Citral, Citronellal, Citronellyloxyacetaldehyd, Cyclamenaldehyd, Lilial und Bourgeonal, zu den Ketonen z.B. die Jonone, alpha-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich Terpene wie Limonen und Pinen. Bevorzugt werden jedoch Mischungen verschiedener Duftstoffe verwendet, die gemeinsam eine ansprechende Duftnote erzeugen.

Solche Parfümöle können auch natürliche Duftstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller-Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie O-rangenblütenöl, Neroliöl, Orangenschalenöl und Sandelholzöl.

Weitere herkömmliche Duftstoffe, die im Rahmen der vorliegenden Erfindung einsetzbar sind, sind beispielsweise die ätherischen Öle wie Angelikawurzelöl, Anisöl, Arnikablütenöl, Basilikumöl, Bayöl, Champacablütenöl, Edeltannenöl, Edeltannenzapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaïvabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl sowie Ambrettolid, Ambroxan, alpha-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, Boisambrene forte, alpha-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-beta-naphthylketon, Methyl-n-nonylacetaldehyd, Methyl-n-nonylketon, Muskon, beta-Naphtholethylether, beta-Naphtholmethylether, Nerol, n-Nonylaldehyd, Nonylalkohol, n-Octylaldehyd, p-Oxy-Acetophenon, Pentadekanolid, beta-Phenylethylalkohol, Phenylacetaldehyd-Dimethyacetal, Phenylessigsäure, Pulegon, Safrol, Salicylsäureisoamylester, Salicylsäuremethylester, Salicylsäurehexylester, Salicylsäurecyclohexylester, Santalol, Sandelice, Skatol, Terpineol, Thymen, Thymol, Troenan, gamma-Undelacton, Vanillin, Veratrumaldehyd, Zimtaldehyd, Zimtalkohol, Zimtsäure, Zimtsäureethylester, Zimtsäurebenzylester, Diphenyloxid, Limonen, Linalool, Linalylacetat und -propionat, Melusat, Menthol, Menthon, Methyl-n-heptenon, Pinen, Phenylacetaldehyd, Terpinylacetat, Citral, Citronellal und Mischungen daraus.

Die Kombination aus (a) ausgewählter Aminoalkohol-Verbindung, (b) Duftstoffaldehyd und/oder Duftstoffketon und (c) ausgewähltem Kieselsäureester wird bevorzugt in Wasch- oder Reinigungsmitteln, Weichspülern oder Kosmetika eingesetzt. Es kann sich dabei um feste, gelförmige oder flüssige Formulierungen handeln, wobei feste Formulierungen in Pulver-, Granulat-, TablettenForm vorliegen können. Bei flüssigen Formulierungen kann es sich um Lösungen, Emulsionen oder Dispersionen handeln. Flüssige Wasch- oder Reinigungsmittel und flüssige Weichspüler enthalten vorzugsweise Wasser als Hauptlösungsmittel.

Typischerweise wird in Endformulierungen, das heißt gebrauchsfertigen Wasch- oder Reinigungsmitteln, Weichspülern oder Kosmetika, die Kombination aus (a) ausgewählter Aminoalkohol-Verbindung, (b) Duftstoffaldehyd und/oder Duftstoffketon und (c) ausgewähltem Kieselsäureester in Mengen von weniger als 10 Gew.-%, vorzugsweise weniger als 5 Gew.-%, insbesondere weniger als 3 Gew.-% eingesetzt.

Bevorzugt wird die Kombination aus (a) ausgewählter Aminoalkohol-Verbindung, (b) Duftstoffaldehyd und/oder Duftstoffketon und (c) ausgewähltem Kieselsäureester in Wasch- oder Reinigungsmitteln oder Weichspülern eingesetzt.

Zusätzlich zu der Kombination aus (a) ausgewählter Aminoalkohol-Verbindung, (b) Duftstoffaldehyd und/oder Duftstoffketon und (c) ausgewähltem Kieselsäureester und den optionalen weiteren Duftstoffen kann das Wasch- oder Reinigungsmittel bzw. kann der Weichspüler weitere Inhaltsstoffe enthalten, die die anwendungstechnischen und/oder ästhetischen Eigenschaften des Waschoder Reinigungsmittels bzw. des Weichspülers weiter verbessern. Im Rahmen der vorliegenden Erfindung enthält das Wasch- oder Reinigungsmittel bzw. der Weichspüler vorzugsweise zusätzlich einen oder mehrere Stoffe aus der Gruppe der Tenside, Gerüststoffe, Bleichmittel, Bleichkatalysatoren, Bleichaktivatoren, Enzyme, Elektrolyte, nichtwässrigen Lösungsmittel, pH-Stellmittel, Parfümzusammensetzungen, Parfümträger, Fluoreszenzmittel, Farbstoffe, Hydrotope, Schauminhibitoren, Silikonöle, Soil-Release-Polymere, Vergrauungsinhibitoren, Einlaufverhinderer, Knitterschutzmittel, Farbübertragungsinhibitoren, weitere antimikrobiellen Wirkstoffe, Germizide, Fungizide, Antioxidantien, Konservierungsmittel, Korrosionsinhibitoren, Antistatika, Bittermittel, Bügelhilfsmittel, Phobier- und Imprägniermittel, Quell- und Schiebefestmittel, weichmachenden Komponenten, hautpflegenden Mittel, Waschkraft-verstärkenden Polymer ("Booster-Polymere") sowie UV-Absorber.

### Beispiel 1

Zur Bestimmung des Abbaus von Schlechtgerüchen wurde eine Haushaltswaschmaschine (Miele W1735) mit 3 kg getragener Wäsche sowie 75 ml eines flüssigen Vollwaschmittels, welches 0,45 Gew.-% Parfüm (bezogen auf das gesamte flüssige Waschmittel) enthielt, beladen. Die Wäsche wurde bei 40°C gewaschen und dann für 4 Tage bei 20°C in der Waschtrommel liegen gelassen (V1). Das Parfüm des flüssigen Vollwaschmittels enthielt Octanal, Cyclamenaldehyd und 2-Methylundecanal.

Zehn olfaktorisch geschulte Personen haben die Wäsche frisch, nach 1 Tag und nach 4 Tagen abgerochen und die Intensität des Schlechtgeruchs auf einer Skala von 1 bis 10 (1 = nicht mehr wahrnehmbar bis 10 = extrem stark) bestimmt. Durch die in der Waschmaschine und im Waschwasser vorhandenen Bakterien sowie dem Eintrag durch die getragene Wäsche entsteht ein Schlechtgeruch.

In einem weiteren Waschversuch (V2) wurden zusätzlich zu dem Vollwaschmittel 0,6 Gew.-% (bezogen auf das gesamte flüssige Waschmittel) eines Kieselsäureestergemisches aus Phenylethylkieselsäureester, Geranylkieselsäureester und Citronellylkieselsäureester zudosiert.

In einem weiteren Waschversuch (V3) wurden zusätzlich zu dem Vollwaschmittel 2 Gew.-% (bezogen auf das gesamte flüssige Waschmittel) eines Aminoalkoholgemisches aus 2-Amino-1,3-propandiol und 2-Amino-2-methyl-1,3-propandiol zudosiert.

In noch einem weiteren Waschversuch (E1) wurden zusätzlich zu dem Vollwaschmittel 0,3 Gew.-% (bezogen auf das gesamte flüssige Waschmittel) eines Kieselsäureestergemisches aus Phenylethylkieselsäureester, Geranylkieselsäureester und Citronellylkieselsäureester und 1 Gew.-% (bezogen auf das gesamte flüssige Waschmittel) eines Aminoalkoholgemisches aus 2-Amino-1,3-propandiol und 2-Amino-2-methyl-1,3-propandiol zudosiert.

Die Ergebnisse sind in Tabelle 1 gezeigt.

**Tabelle 1: Intensität des Schlechtgeruchs**

| | V1 | V2 | V3 | E1 |
|---|---|---|---|---|
| Frisch | 2 | 2,2 | 2,5 | 2,3 |
| 1 Tag | 5,7 | 4,5 | 3,3 | 2,5 |
| 4 Tage | 8,1 | 7,4 | 4 | 2,3 |

Die Ergebnisse zeigen die deutlich verbesserte Leistung der Kombination aus (a) einer ausgewählten Aminoalkohol-Verbindung der allgemeinen Formel (I), (b) einem Duftstoffaldehyd und/oder Duftstoffketon und (c) einem ausgewählten Kieselsäureester beim Abbau von Schlechtgerüchen, insbesondere über einen Zeitraum von mehreren Tagen.

### Beispiel 2

Ein Textil aus Baumwollfrottee (Größe: 30 x 30 cm) wurde mit 100 mg des Schlechtgeruchs "Schweiß" (20 Gew.-% Octansäure, 20 Gew.-% Nonansäure, 20 Gew.-% 3-Methylbutansäure, 2-Ethyl-2-hexensäure und 20 Gew.-% 3-Mercapto-1-hexanol) zwangsappliziert.

Anschließend wurde eine Haushaltswaschmaschine (Miele W1735) mit 3,5 kg Begleitwäsche sowie dem zwangsapplizierten Stofflappen beladen. Zusätzlich wurden 75 ml eines flüssigen Vollwaschmittels, welches 0,45 Gew.-% Parfüm (bezogen auf das gesamte flüssige Waschmittel) enthielt, zugegeben. Die Wäsche wurde bei 40°C gewaschen und dann für 4 Tage bei 20°C in der Waschtrommel liegen gelassen (V1). Das Parfüm des flüssigen Vollwaschmittels enthielt Octanal, Cyclamenaldehyd und 2-Methylundecanal.

Außerdem wurden Waschversuche durchgeführt, bei denen die Waschmaschine zusätzlich zu dem Vollwaschmittel
- mit 0,6 Gew.-% (bezogen auf das gesamte flüssige Waschmittel) eines Kieselsäureestergemisches aus Phenylethylkieselsäureester, Geranylkieselsäureester und Citronellylkieselsäureester (V2),
- 2 Gew.-% (bezogen auf das gesamte flüssige Waschmittel) eines Aminoalkoholgemisches aus 2-Amino-1,3-propandiol und 2-Amino-2-methyl-1,3-propandiol (V3) und
- 0,3 Gew.-% (bezogen auf das gesamte flüssige Waschmittel) eines Kieselsäureestergemisches aus Phenylethylkieselsäureester, Geranylkieselsäureester und Citronellylkieselsäureester und 1 Gew.-% (bezogen auf das gesamte flüssige Waschmittel)eines Aminoalkoholgemisches aus 2-Amino-1,3-propandiol und 2-Amino-2-methyl-1,3-propandiol (E1)
zudosiert wurden.

Die Ergebnisse sind in Tabelle 2 gezeigt.

**Tabelle 2: Intensität des Schlechtgeruchs**

| | V1 | V2 | V3 | E1 |
|---|---|---|---|---|
| Frisch | 9 | 8,6 | 5,7 | 5 |
| 1 Tag | 7,4 | 7,1 | 3,3 | 2,5 |
| 4 Tage | 6,8 | 6,4 | 2,9 | 2,3 |

Die Ergebnisse zeigen die deutlich verbesserte Leistung der erfindungsgemäßen Kombination der Kombination aus (a) einer ausgewählten Aminoalkohol-Verbindung der allgemeinen Formel (I), (b) einem Duftstoffaldehyd und/oder Duftstoffketon und (c) einem ausgewählten Kieselsäureester beim Abbau von Schlechtgerüchen, besonders des Schlechtgeruchs "Schweiß", insbesondere über einen Zeitraum von mehreren Tagen.

## Patentansprüche

1. Kombination aus
(a) einer Aminoalkohol-Verbindung der allgemeinen Formel (I) 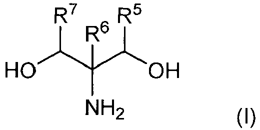 wobei R⁵, R⁶, R⁷ unabhängig voneinander für H oder einen Kohlenwasserstoff-Rest stehen, der acyclisch oder cyclisch, substituiert oder unsubstituiert, verzweigt oder unverzweigt sowie gesättigt oder ungesättigt sein kann, oder Gemische dieser Verbindungen,
(b) einem Duftstoffaldehyd und/oder Duftstoffketon und
(c) einem nicht-vollständig mit Duftstoffalkoholen umgeesterten Kieselsäureester der allgemeinen Formel (II): 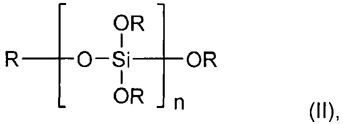 wobei alle R unabhängig voneinander ausgewählt sind aus der Gruppe, die H, die geradkettigen oder verzweigten, gesättigten oder ungesättigten, substituierten oder unsubstituierten C₁₋₆-Kohlenwasserstoffreste und die Duftstoffalkoholreste enthält, und n Werte aus dem Bereich 2 bis 100 annimmt und
wobei mindestens 10 mol-%, vorzugsweise mindestens 20 mol-% und insbesondere bevorzugt sogar mehr als 40 mol-% der Reste R der allgemeinen Formel (II) ausgewählt sind aus der Gruppe bestehend aus den Resten der Duftstoffalkohole 10-Undecen-1-ol, 2,6-Dimethylheptan-2-ol, 2-Methylbutanol, 2-Methylpentanol, 2-Phenoxyethanol, 2-Phenylpropanol, 2-tert-Butyl-cyclohexanol, 3,5,5-Trimethylcyclohexanol, 3-Hexanol, 3-Methyl-5-phenylpentanol, 3-Octanol, 3-Phenylpropanol, 4-Heptenol, 4-Isopropylcyclohexanol, 4-tert-Butylcyclohexanol, 6,8-Dimethyl-2-nonanol, 6-Nonen-1-ol, 9-Decen-1-ol, alpha-Methylbenzylalkohol, alpha-Terpineol, Amylsalicylat, Benzylalkohol, Benzylsalicylat, beta-Terpineol, Butylsalicylat, Citronellol, Cyclohexylsalicylat, Decanol, Dihydromyrcenol, Dimethylbenzylcarbinol, Dimethylheptanol, Dimethyloctanol, Ethylsalicylat, Ethylvanillin, Eugenol, Farnesol, Geraniol, Heptanol, Hexylsalicylat, Isoborneol, Isoeugenol, Isopulegol, Linalool, Menthol, Myrtenol, n-Hexanol, Nerol, Nonanol, Octanol, p-Menthan-7-ol, Phenylethylalkohol, Phenol, Phenylsalicylat, Tetrahydrogeraniol, Tetrahydrolinalool, Thymol, trans-2-cis-6-Nonadicnol, trans-2-Nonen-1-ol, trans-2-Octenol, Undecanol, Vanillin, Zimtalkohol und Mischungen daraus.

2. Kombination nach Anspruch 1, **dadurch gekennzeichnet, dass** der Duftstoffaldehyd und/oder das Duftstoffketon zumindest teilweise unter Ringschluss mit der Aminoalkohol-Verbindung der allgemeinen Formel (I) zu einer 1-Aza-3,7-dioxabicyclo[3.3.0]octan-Verbindung der allgemeinen Formel (III) 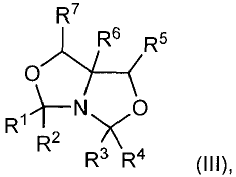 wobei R¹, R², R³, R⁴ unabhängig voneinander für Reste stehen, die in einer Verbindung der allgemeinen Formel R¹-C(=O)-R² oder R³-C(=O)-R⁴ einen Duftstoffaldehyd mit mindestens 6 Kohlenstoffatomen oder ein Duftstoffketon mit mindestens 6 Kohlenstoffatomen ergeben, reagiert.

3. Kombination nach Anspruch 2, **dadurch gekennzeichnet, dass** R², R⁴, R⁵, R⁶, R⁷ Wasserstoff bedeuten und R¹ und R³ jeweils einen C₅₋₂₄-Kohlenwasserstoffrest bedeuten.

4. Kombination nach einem der Ansprüche 1 bis 3, **dadurch gekennzeichnet, dass** die Duftstoffaldehyde und die Duftstoffketone ausgewählt sind aus den Jasmonen, lononen, Damasconen und Damascenonen, Menthon, Carvon, Iso-E-Super, Methylheptenon, Melonal, Cymal, Ethylvanillin, Helional, Hydroxycitronellal, Koavon, Methyl-nonyl-acetaldehyd, Phenylacetaldehyd, Undecylenaldehyd, 3-Dodecen-1-al, alpha-n-Amylzimtaldehyd, Benzaldehyd, 3-(4-tert-Butylphenyl)-propanal, 2-Methyl-3-(para-methoxyphenylpropanal), 2-Methyl-4-(2,6,6-trimethyl-2(1)-cyclohexen-1-yl)butanal, 3-Phenyl-2-propenal, cis-/trans-3,7-Dimethyl-2,6-octadien-I-al, 3,7-Dimethyl-6-octen-I-al, [(3,7-Dimethyl-6-octenyl)oxy]acetaldehyd, 4-Iso-propylbenzyaldehyd, 2,4-Dimethyl-3-cyclohexen-1-carboxyaldehyd, 2-Methyl-3-(isopropylphenyl)propanal, Decylaldehyd, 2,6-Dimethyl-5-heptenal, alpha-n-Hexylzimtaldehyd, 7-Hydroxy-3,7-dimethyloctanal, Undecenal, 2,4,6-Trimethyl-3-cyclohexene-I-carboxaldehyd, 1-Dodecanal, 2,4-Dimethylcyclohexene-3-carboxaldehyd, 4-(4-Hydroxy-4-methyl pentyl)-3-cylohexene-I-carboxaldehyd, 2-Methyl undecanal, 2-Methyldecanal, 1-nonanal, 1-Octanal, 2,6,10-Trimethyl-5,9-undecadienal, 2-Methyl-3-(4-tertbutyl)propanal, Dihydrozimtaldehyd, 3,7-Dimethyloctan-1-al, 1-Undecanal, 10-Undecen-1-al, 4-Hydroxy-3-methoxybenzaldehyd, trans-4-decenal, 2,6-Nonadienal, para-Tolylacetaldehyd, 3,7-Dimethyl-2-methylene-6-octenal, 2-Methyloctanal, alpha-Methyl-4-(1-methylethyl)benzeneacetaldehyd, 2-methyl-3-phenyl-2-propen-1-al, 3,5,5-Trimethylhexanal, 3-Propyl-bicyclo[2.2.1]hept-5-ene-2-carbaldehyd, 9-Decenal, 3-Methyl-5-phenyl-1-pentanal, Methylnonylacetaldehyd, Citral, 1-Decanal, Florhydral, 2,4-Dimethyl-3-cyclohexen-1-carboxaldehyd, Heliotropin und Mischungen daraus.

5. Kombination gemäß einem der Ansprüche 1 bis 4, **dadurch gekennzeichnet, dass** der Duftstoffaldehyd und/oder das Duftstoffketon zumindest teilweise unter Ringschluss mit der Aminoalkohol-Verbindung der allgemeinen Formel (I) zu einer Verbindung der allgemeinen Formel (IV) 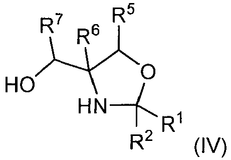 wobei R¹ und R² unabhängig voneinander für Reste stehen, die in einer Verbindung der allgemeinen Formel R¹-C(=O)-R² einen Duftstoffaldehyd mit mindestens 6 Kohlenstoffatomen oder ein Duftstoffketon mit mindestens 6 Kohlenstoffatomen ergeben, reagiert.

6. Kombination gemäß einem der Ansprüche 1 bis 5, **dadurch gekennzeichnet, dass** ein Teil der Reste R der allgemeinen Formel (II), vorzugsweise mindestens 5 mol-% der Reste R ausgewählt sind aus der Gruppe Methyl, Ethyl, n-Propyl, iso-Propyl, n-Butyl, iso-Butyl und tert-Butyl.

7. Kombination gemäß einem der Ansprüche 1 bis 6, **dadurch gekennzeichnet, dass** n Werte aus dem Bereich von 2 bis 50, vorzugsweise aus dem Bereich von 2 bis 20 und insbesondere aus dem Bereich von 3 bis 10 unter besonderer Bevorzugung der Werte 4, 5, 6, 7 und 8, annimmt.

8. Verwendung der Kombination nach einem der Ansprüche 1 bis 7 als Duftstoffvorläufer.

9. Verwendung nach Anspruch 8, **dadurch gekennzeichnet, dass** die Kombination zusammen mit anderen Duftstoffen eingesetzt wird.

10. Wasch- oder Reinigungsmittel, Weichspüler oder Kosmetika, enthaltend eine Kombination aus
(a) einer Aminoalkohol-Verbindung der allgemeinen Formel (I) 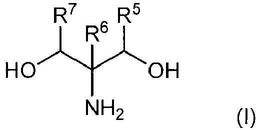 wobei R⁵, R⁶, R⁷ unabhängig voneinander für H oder einen Kohlenwasserstoff-Rest stehen, der acyclisch oder cyclisch, substituiert oder unsubstituiert, verzweigt oder unverzweigt sowie gesättigt oder ungesättigt sein kann, oder Gemische dieser Verbindungen,
(b) einem Duftstoffaldehyd und/oder Duftstoffketon und
(c) einem Kieselsäureester der allgemeinen Formel (II): 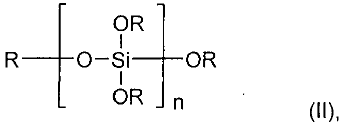 wobei alle R unabhängig voneinander ausgewählt sind aus der Gruppe, die H, die geradkettigen oder verzweigten, gesättigten oder ungesättigten, substituierten oder unsubstituierten C₁₋₆-Kohlenwasserstoffreste und die Duftstoffalkoholreste enthält, und n Werte aus dem Bereich 2 bis 100 annimmt und
wobei mindestens 10 mol-%, vorzugsweise mindestens 20 mol-% und insbesondere bevorzugt sogar mehr als 40 mol-% der Reste R der allgemeinen Formel (II) ausgewählt sind aus der Gruppe bestehend aus den Resten der Duftstoffalkohole 10-Undecen-1-ol, 2,6-Dimethylheptan-2-ol, 2-Methylbutanol, 2-Methylpentanol, 2-Phenoxyethanol, 2-Phenylpropanol, 2-tert-Butyl-cyclohexanol, 3,5,5-Trimethylcyclohexanol, 3-Hexanol, 3-Methyl-5-phenylpentanol, 3-Octanol, 3-Phenylpropanol, 4-Heptenol, 4-Isopropylcyclohexanol, 4-tert-Butylcyclohexanol, 6,8-Dimethyl-2-nonanol, 6-Nonen-1-ol, 9-Decen-1-ol, alpha-Methylbenzylalkohol, alpha-Terpineol, Amylsalicylat, Benzylalkohol, Benzylsalicylat, beta-Terpineol, Butylsalicylat, Citronellol, Cyclohexylsalicylat, Decanol, Dihydromyrcenol, Dimethylbenzylcarbinol, Dimethylheptanol, Dimethyloctanol, Ethylsalicylat, Ethylvanillin, Eugenol, Farnesol, Geraniol, Heptanol, Hexylsalicylat, Isoborneol, Isoeugenol, Isopulegol, Linalool, Menthol, Myrtenol, n-Hexanol, Nerol, Nonanol, Octanol, p-Menthan-7-ol, Phenylethylalkohol, Phenol, Phenylsalicylat, Tetrahydrogeraniol, Tetrahydrolinalool, Thymol, trans-2-cis-6-Nonadicnol, trans-2-Nonen-1-ol, trans-2-Octenol, Undecanol, Vanillin, Zimtalkohol und Mischungen daraus.

11. Verfahren zur Verlängerung des Duftempfindens von Wasch- oder Reinigungsmitteln, Weichspülern oder Kosmetika oder mit diesen behandelten festen Oberflächen, **dadurch gekennzeichnet, dass** man den Wasch- oder Reinigungsmitteln, Weichspülern oder Kosmetika die Kombination nach einem der Ansprüche 1 bis 7 zusetzt, wobei der Duftstoff durch Hydrolyse wieder freigesetzt wird.

12. Verfahren zum Abbau von Schlechtgerüchen durch Einsatz einer Kombination aus
(a) einer Aminoalkohol-Verbindung der allgemeinen Formel (I) 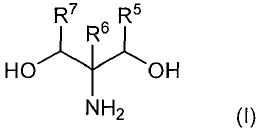 wobei R⁵, R⁶, R⁷ unabhängig voneinander für H oder einen Kohlenwasserstoff-Rest stehen, der acyclisch oder cyclisch, substituiert oder unsubstituiert, verzweigt oder unverzweigt sowie gesättigt oder ungesättigt sein kann, oder Gemische dieser Verbindungen,
(b) einem Duftstoffaldehyd und/oder Duftstoffketon und
(c) einem Kieselsäureester der allgemeinen Formel (II): 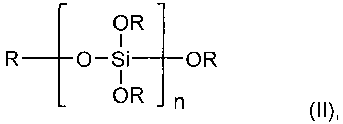 wobei alle R unabhängig voneinander ausgewählt sind aus der Gruppe, die H, die geradkettigen oder verzweigten, gesättigten oder ungesättigten, substituierten oder unsubstituierten C₁₋₆-Kohlenwasserstoffreste und die Duftstoffalkoholreste enthält, und n Werte aus dem Bereich 2 bis 100 annimmt und
wobei mindestens 10 mol-%, vorzugsweise mindestens 20 mol-% und insbesondere bevorzugt sogar mehr als 40 mol-% der Reste R der allgemeinen Formel (II) ausgewählt sind aus der Gruppe bestehend aus den Resten der Duftstoffalkohole 10-Undecen-1-ol, 2,6-Dimethylheptan-2-ol, 2-Methylbutanol, 2-Methylpentanol, 2-Phenoxyethanol, 2-Phenylpropanol, 2-tert-Butyl-cyclohexanol, 3,5,5-Trimethylcyclohexanol, 3-Hexanol, 3-Methyl-5-phenylpentanol, 3-Octanol, 3-Phenylpropanol, 4-Heptenol, 4-Isopropylcyclohexanol, 4-tert-Butylcyclohexanol, 6,8-Dimethyl-2-nonanol, 6-Nonen-1-ol, 9-Decen-1-ol, alpha-Methylbenzylalkohol, alpha-Terpineol, Amylsalicylat, Benzylalkohol, Benzylsalicylat, beta-Terpineol, Butylsalicylat, Citronellol, Cyclohexylsalicylat, Decanol, Dihydromyrcenol, Dimethylbenzylcarbinol, Dimethylheptanol, Dimethyloctanol, Ethylsalicylat, Ethylvanillin, Eugenol, Farnesol, Geraniol, Heptanol, Hexylsalicylat, Isoborneol, Isoeugenol, Isopulegol, Linalool, Menthol, Myrtenol, n-Hexanol, Nerol, Nonanol, Octanol, p-Menthan-7-ol, Phenylethylalkohol, Phenol, Phenylsalicylat, Tetrahydrogeraniol, Tetrahydrolinalool, Thymol, trans-2-cis-6-Nonadicnol, trans-2-Nonen-1-ol, trans-2-Octenol, Undecanol, Vanillin, Zimtalkohol und Mischungen daraus.

## Claims

1. Combination of
(a) an amino alcohol compound of the general Formula (I) 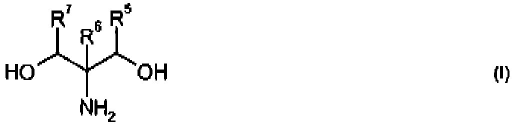 wherein R⁵, R⁶, R⁷ independently of each other stand for H or a hydrocarbon group that can be acyclic or cyclic, substituted or unsubstituted, branched or unbranched as well as saturated or unsaturated, or mixtures of these compounds,
(b) a scent aldehyde and/or scent ketone and
(c) a silicic acid ester of the general Formula (II), incompletely transesterified with scent alcohols: 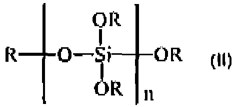 wherein all R, independently of each other, are selected from the group that comprises H, the linear or branched, saturated or unsaturated, substituted or unsubstituted C₁₋₆ hydrocarbon groups and the scent alcohol groups, and n assumes values in the range 2 to 100 and wherein at least 10 mol %, preferably at least 20 mol % and particularly preferably even more than 40 mol % of the R groups of the general Formula (II) are selected from the group consisting of the residues of the scent alcohols 10-undecen-1-ol, 2,6-dimethylheptan-2-ol, 2-methylbutanol, 2-methylpentanol, 2-phenoxyethanol, 2-phenylpropanol, 2-*tert-*butylcyclohexanol, 3,5,5-trimethylcyclohexanol, 3-hexanol, 3-ethyl-5-phenylpentanol, 3-octanol, 3-phenylpropanol, 4-heptenol, 4-isopropylcyclohexanol, 4-*tert*-butylcyclohexanol, 6,8-dimethyl-2-nonanol, 6-nonen-1-ol, 9-decen-1-ol, alpha-methylbenzyl alcohol, alpha-terpineol, amyl salicylate, benzyl alcohol, benzyl salicylate, beta-terpineol, butyl salicylate, citronellol, cyclohexyl salicylate, decanol, dihydromyrcenol, dimethylbenzyl carbinol, dimethylheptanol, dimethyloctanol, ethyl salicylate, ethyl vanillin, eugenol, farnesol, geraniol, heptanol, hexyl salicylate, *iso*borneol, *iso*eugenol, *iso*pulegol, linalool, menthol, myrtenol, *n*-hexanol, nerol, nonanol, octanol, *p-*menthan-7-ol, phenylethyl alcohol, phenol, phenyl salicylate, tetrahydrogeraniol, tetrahydrolinalool, thymol, *trans*-2-cis-6-nonadienol, *trans-*2-nonen-1-ol, *trans-*2-octenol, undecanol, vanillin, cinnamyl alcohol and mixtures thereof.

2. Combination according to claim 1, **characterised in that** the scent aldehyde and/or the scent ketone reacts at least partially under cyclisation with the amino alcohol compound of the general Formula (I) to form a 1-aza-3,7-dioxabicyclo[3.3.0]octane compound of the general Formula (III) 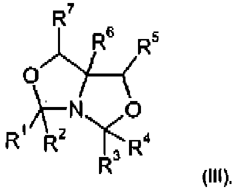 wherein R¹, R², R³, R⁴ independently of each other stand for groups that in a compound of the general Formula R¹-C(=O)-R² or R³-C(=O)-R⁴ form a scent aldehyde containing at least 6 carbon atoms or a scent ketone containing at least 6 carbon atoms.

3. Combination according to claim 2, **characterised in that** R², R⁴, R⁵, R⁶, R⁷ mean hydrogen and R¹ and R³ each mean a C₅₋₂₄ hydrocarbon group.

4. Combination according to one of claims 1 to 3, **characterised in that** the scent aldehydes and the scent ketones are selected from the jasmones, ionones, damascones and damascenones, menthone, carvone, iso-E-super, methylheptenone, melonal, cymal, ethyl vanillin, helional, hydroxycitronellal, koavone, methylnonylacetaldehyde, phenylacetaldehyde, undecylenaldehyde, 3-dodecen-1-al, alpha-*n*-amylcinnamaldehyde, benzaldehyde, 3-(4-*tert-*butylphenyl)-propanal, 2-methyl-3-(*para*-methoxyphenylpropanal), 2-methyl-4-(2,6,6-trimethyl-2(1)-cyclohexen-1-yl)butanal, 3-phenyl-2-propenal, *cis*/*trans-*3,7-dimethyl-2,6-octadien-1-al, 3,7-dimethyl-6-octen-1-al, [(3,7-dimethyl-6-octenyl)oxy]acetaldehyde, 4-isopropylbenzyaldehyde, 2,4-dimethyl-3-cyclohexene-1-carboxyaldehyde, 2-methyl-3-(isopropylphenyl)propanal, decylaldehyde, 2,6-dimethyl-5-heptenal, alpha-*n*-hexylcinnamaldehyde, 7-hydroxy-3,7-dimethyloctanal, undecenal, 2,4,6-trimethyl-3-cyclohexene-1-carboxaldehyde, 1-dodecanal, 2,4-dimethylcyclohexene-3-carboxaldehyde, 4-(4-hydroxy-4-methylpentyl)-3-cylohexene-1-carboxaldehyd, 2-methylundecanal, 2-methyldecanal, 1-nonanal, 1-octanal, 2,6,10-trimethyl-5,9-undecadienal, 2-methyl-3-(4-*tert*-butyl)propanal, dihydrocinnamaldehyde, 3,7-dimethyloctan-1-al, 1-undecanal, 10-undecen-1-al, 4-hydroxy-3-methoxybenzaldehyde, *trans*-4-decenal, 2,6-nonadienal, *para*-tolylacetaldehyde, 3,7-dimethyl-2-methylene-6-octenal, 2-methyloctanal, alpha-methyl-4-(1-methylethyl)benzeneacetaldehyde, 2-methyl-3-phenyl-2-propen-1-al, 3,5,5-trimethylhexanal, 3-propyl-bicyclo[2.2.1]hept-5-ene-2-carbaldehyde, 9-decenal, 3-methyl-5-phenyl-1-pentanal, methylnonylacetaldehyde, citral, 1-decanal, florhydral, 2,4-dimethyl-3-cyclohexene-1-carboxaldehyde, heliotropin and mixtures thereof.

5. Combination according to one of claims 1 to 4, **characterised in that** the scent aldehyde and/or the scent ketone reacts at least partially under cyclisation with the amino alcohol compound of the general Formula (I) to form a compound of the general Formula (IV) 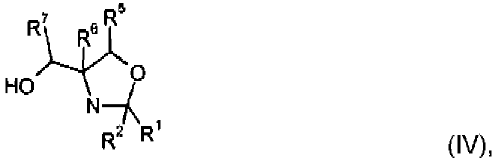 wherein R¹ and R², independently of one another stand for groups that in a compound of the general Formula R¹-C(=O)-R² form a scent aldehyde containing at least 6 carbon atoms or a scent ketone containing at least 6 carbon atoms.

6. Combination according to one of claims 1 to 5, **characterised in that** a part of the R groups of the general Formula (II), preferably at least 5 mol % of the R groups, are selected from the group methyl, ethyl, *n*-propyl, isopropyl, *n-*butyl, isobutyl and *tert*-butyl.

7. Combination according to one of claims 1 to 6, **characterised in that** n assumes values in the range 2 to 50, preferably in the range 2 to 20 and in particular in the range 3 to 10, with particular preference assumes the values 4, 5, 6, 7 or 8.

8. Use of the combination according to one of claims 1 to 7 as a scent precursor.

9. Use according to claim 8, **characterised in that** the combination is employed together with other scents.

10. Washing or cleaning agent, fabric softener or cosmetics, comprising a combination of
(a) an amino alcohol compound of the general Formula (I) 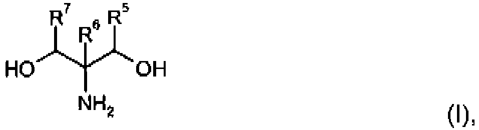 wherein R⁵, R⁶, R⁷ independently of each other stand for H or a hydrocarbon group that can be acyclic or cyclic, substituted or unsubstituted, branched or unbranched as well as saturated or unsaturated,
or mixtures of these compounds,
(b) a scent aldehyde and/or scent ketone and
(c) a silicic acid ester of the general Formula (II): 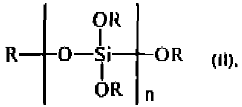 wherein all R, independently of each other, are selected from the group that comprises H, the linear or branched, saturated or unsaturated, substituted or unsubstituted C₁₋₆ hydrocarbon groups and the scent alcohol groups, and n assumes values in the range 2 to 100 and wherein at least 10 mol %, preferably at least 20 mol % and particularly preferably even more than 40 mol % of the R groups of the general Formula (II) are selected from the group consisting of the residues of the scent alcohols 10-undecen-1-ol, 2,6-dimethylheptan-2-ol, 2-methylbutanol, 2-methylpentanol, 2-phenoxyethanol, 2-phenylpropanol, 2-*tert*-butylcyclohexanol, 3,5,5-trimethylcyclohexanol, 3-hexanol, 3-methyl-5-phenylpentanol, 3-octanol, 3-phenylpropanol, 4-heptenol, 4-isopropylcyclohexanol, 4-*tert*-butylcyclohexanol, 6,8-dimethyl-2-nonanol, 6-nonen-1-ol, 9-decen-1-ol, alpha-methylbenzyl alcohol, alpha-terpineol, amyl salicylate, benzyl alcohol, benzyl salicylate, beta-terpineol, butyl salicylate, citronellol, cyclohexyl salicylate, decanol, dihydromyrcenol, dimethylbenzyl carbinol, dimethylheptanol, dimethyloctanol, ethyl salicylate, ethyl vanillin, eugenol, farnesol, geraniol, heptanol, hexyl salicylate, *iso*borneol, *iso*eugenol, *iso*pulegol, linalool, menthol, myrtenol, *n-*hexanol, nerol, nonanol, octanol, *p*-menthan-7-ol, phenylethyl alcohol, phenol, phenyl salicylate, tetrahydrogeraniol, tetrahydrolinalool, thymol, *trans-*2*-cis-*6-nonadienol, *trans*-2-nonen-1-ol, *trans-*2-octenol, undecanol, vanillin, cinnamyl alcohol and mixtures thereof.

11. Method for extending the perceived fragrance of washing or cleaning agents, fabric softeners or cosmetics or of hard surfaces treated with them, **characterised in that** the combination according to one of claims 1 to 7 is added to the washing or cleaning agents, fabric softeners or cosmetics, wherein the scent is released by hydrolysis.

12. Method for eliminating malodours by adding a combination of
(a) an amino alcohol compound of the general Formula (I) 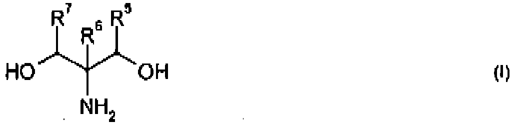 wherein R⁵, R⁶, R⁷ independently of each other stand for H or a hydrocarbon group that can be acyclic or cyclic, substituted or unsubstituted, branched or unbranched as well as saturated or unsaturated, or mixtures of these compounds,
(b) a scent aldehyde and/or scent ketone and
(c) a silicic acid ester of the general Formula (II): 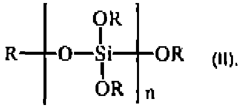 wherein all R, independently of each other, are selected from the group that comprises H, the linear or branched, saturated or unsaturated, substituted or unsubstituted C₁₋₆ hydrocarbon groups and the scent alcohol groups, and n assumes values in the range 2 to 100 and wherein at least 10 mol %, preferably at least 20 mol % and particularly preferably even more than 40 mol % of the R groups of the general Formula (II) are selected from the group consisting of the residues of the scent alcohols 10-undecen-1-ol, 2,6-dimethylheptan-2-ol, 2-methylbutanol, 2-methylpentanol, 2-phenoxyethanol, 2-phenylpropanol, 2-*tert*-butylcyclohexanol, 3,5,5-trimethylcyclohexanol, 3-hexanol, 3-methyl-5-phenylpentanol, 3-octanol, 3-phenylpropanol, 4-heptenol, 4-isopropylcyclohexanol, 4-*tert*-butylcyclohexanol, 6,8-dimethyl-2-nonanol, 6-nonen-1-ol, 9-decen-1-ol, alpha-methylbenzyl alcohol, alpha-terpineol, amyl salicylate, benzyl alcohol, benzyl salicylate, beta-terpineol, butyl salicylate, citronellol, cyclohexyl salicylate, decanol, dihydromyrcenol, dimethylbenzyl carbinol, dimethylheptanol, dimethyloctanol, ethyl salicylate, ethyl vanillin, eugenol, farnesol, geraniol, heptanol, hexyl salicylate, isoborneol, *iso*eugenol, *iso*pulegol, linalool, menthol, myrtenol, *n-*hexanol, nerol, nonanol, octanol, *p*-menthan-7-ol, phenylethyl alcohol, phenol, phenyl salicylate, tetrahydrogeraniol, tetrahydrolinalool, thymol, *trans-2-cis-6-*nonadienol, *trans*-2-nonen-1-ol, *trans*-2-octenol, undecanol, vanillin, cinnamyl alcohol and mixtures thereof

## Revendications

1. Combinaison entre
(a) un composé de type aminoalcool répondant à la formule générale (I) 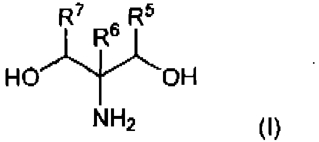 dans laquelle R⁵, R⁶, R⁷ représentent, indépendamment l'un de l'autre, H ou un résidu hydrocarboné lequel peut être acyclique ou cyclique, substitué ou non-substitué, ramifié ou linéaire ainsi que saturé ou insaturé, ou des mélanges de ces composés,
(b) un aldéhyde odorant et/ou une cétone odorante, et
(c) un ester d'acide silicique répondant à la formule générale (II) lequel a subi une transestérification incomplète avec des alcools odorants : 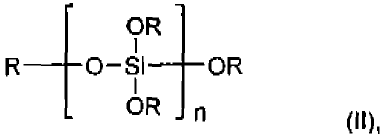 dans laquelle tous les R sont choisis, indépendamment l'un de l'autre, dans le groupe contenant H, les résidus hydrocarbonés en C₁₋₆ linéaires ou ramifiés, saturés ou insaturés, substitués ou non-substitués, et les résidus d'alcools odorants, et n correspond à des valeurs comprises entre 2 et 100, et
au moins 10 % en mol, de préférence au moins 20 % en mol, et avec une préférence particulière même plus de 40 % en mol, des résidus R de la formule générale (II) étant choisis dans le groupe constitué des résidus des alcools odorants 10-undécén-1-ol, 2,6-diméthylheptan-2-ol, 2-méthylbutanol, 2-méthylpentanol, 2-phénoxy-éthanol, 2-phénylpropanol, 2-tert-butyl-cyclohexanol, 3,5,5-triméthyl-cyclohexanol, 3-hexanol, 3-méthyl-5-phenylpentanol, 3-octanol, 3-phénylpropanol, 4-hepténol, 4-isopropylcyclohexanol, 4-tert-butylcyclohexanol, 6,8-diméthyl-2-nonanol, 6-nonén-1-ol, 9-décén-1-ol, alcool alpha-méthylbenzylique, alpha-terpinéol, salicylate d'amyle, alcool benzylique, salicylate de benzyle, bêta-terpinéol, salicylate de butyle, citronellol, salicylate de cyclohexyle, décanol, dihydro-myrcénol, diméthylbenzylcarbinol, diméthylheptanol, diméthyloctanol, salicylate d'éthyle, éthylvanilline, eugénol, farnésol, géraniol, heptanol, salicylate d'hexyle, isobornéol, isoeugénol, isopulégol, linalool, menthol, myrténol, n-hexanol, nérol, nonanol, octanol, p-menthane-7-ol, phényléthanol, phénol, salicylate de phényle, tétrahydrogéraniol, tétrahydrolinalool, thymol, trans-2-cis-6-nona-diénol, trans-2-nonén-1-ol, trans-2-octénol, undécanol, vanilline, alcool cinnamique et de leurs mélanges.

2. Combinaison selon la revendication 1, **caractérisée en ce que** ledit aldéhyde odorant et/ou ladite cétone odorante réagit, au moins partiellement en subissant une cyclisation, avec le composé de type aminoalcool répondant à la formule générale (I) pour former un composé 1-aza-3,7-dioxabicyclo[3.3.0]octane répondant à la formule générale (III) 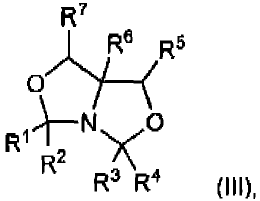 dans laquelle R¹, R², R³, R⁴ représentent, indépendamment l'un de l'autre, des résidus faisant en sorte qu'un composé répondant à la formule générale R¹-C(=O)-R² ou R³-C(=O)-R⁴ soit un aldéhyde odorant renfermant au moins 6 atomes de carbone ou une cétone odorante renfermant au moins 6 atomes de carbone.

3. Combinaison selon la revendication 2, **caractérisée en ce que** R² R⁴, R⁵, R⁶, R⁷ représentent hydrogène et R¹ et R³ représentent chacun un résidu hydrocarboné en C₅₋₂₄.

4. Combinaison selon l'une des revendications 1 à 3, **caractérisée en ce que** les aldéhydes odorants et les cétones odorantes sont choisis parmi les jasmones, les ionones, les damascones et damascénones, la menthone, la carvone, l'iso-E-super, la méthylheptenone, le mélonal, le cymal, l'éthylvanilline, l'hélional, l'hydroxycitronellal, la koavone, le méthyl-nonyl-acétaldéhyde, le phénylacétaldéhyde, l'undécylène-aldéhyde, le 3-dodécén-1-al, l'aldéhyde alpha-n-amylcinnamique, le benzaldéhyde, le 3-(4-tert-butylphényl)-propanal, le 2-méthyl-3-(para-méthoxyphényl-propanal), le 2-méthyl-4-(2,6,6-triméthyl-2(1)-cyclohexén-1-yl)butanal, le 3-phényl-2-propénal, le cis-/trans-3,7-diméthyl-2,6-octadién-1-al, le 3,7-diméthyl-6-octén-1-al, le [(3,7-diméthyl-6-octényl)oxy]acétaldéhyde, le 4-iso-propylbenzyaldéhyde, le 2,4-diméthyl-3-cyclohexène-1 -carboxy-aldéhyde, le 2-méthyl-3-(isopropylphényl)propanal, le décylaldéhyde, le 2,6-diméthyl-5-heptén-al, l'aldéhyde alpha-n-hexylcinnamique, le 7-hydroxy-3,7-diméthyloctanal, l'undécénal, le 2,4,6-triméthyl-3-cyclo-hexène-1-carboxaldéhyde, le 1-dodecanal, le 2,4-diméthylcyclohexène-3-carboxaldéhyde, le 4-(4-hydroxy-4-méthylpentyl)-3-cyclohexène-1-carboxaldéhyde, le 2-méthylundécanal, le 2-méthyldecanal, le 1-nonanal, le 1-octanal, le 2,6,10-triméthyl-5,9-undecadiénal, le 2-méthyl-3-(4-tertbutyl)propanal, l'aldéhyde dihydrocinnamique, le 3,7-diméthyl-1-octanal, le 1-undécanal, le 10-undécén-1-al, le 4-hydroxy-3-méthoxybenzaldéhyde, le trans-4-décénal, le 2,6-nonadiénal, le para-tolylacétaldéhyde, le 3,7-diméthyl-2-méthylène-6-octénal, le 2-méthyloctanal, l'alpha-méthyl-4-(1-méthyléthyl)benzèneacétaldéhyde, le 2-méthyl-3-phényl-2-propén-1-al, le 3,5,5-triméthylhexanal, le 3-propyl-bicyclo[2.2.1]hept-5-ène-2-carbaldéhyde, le 9-décénal, le 3-méthyl-5-phényl-1-pentanal, le méthylnonylacétaldéhyde, le citral, le 1-décanal, le florhydral, le 2,4-diméthyl-3-cyclohexène-1-carboxaldéhyde, l'héliotropine et parmi leurs mélanges.

5. Combinaison selon l'une des revendications 1 à 4, **caractérisée en ce que** ledit aldéhyde odorant et/ou ladite cétone odorante réagit, au moins partiellement en subissant une cyclisation, avec le composé de type aminoalcool répondant à la formule générale (I) pour former un composé répondant à la formule générale (IV) 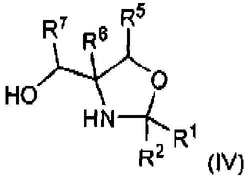 dans laquelle R¹ et R² représentent, indépendamment l'un de l'autre, des résidus faisant en sorte qu'un composé répondant à la formule générale R¹-C(=O)-R² soit un aldéhyde odorant renfermant au moins 6 atomes de carbone ou une cétone odorante renfermant au moins 6 atomes de carbone.

6. Combinaison selon l'une des revendications 1 à 5, **caractérisée en ce qu'**une partie des résidus R répondant à la formule générale (II), de préférence au moins 5 % en mol des résidus R, est choisie dans le groupe méthyle, éthyle, n-propyle, isopropyle, n-butyle, isobutyle et tert-butyle.

7. Combinaison selon l'une des revendications 1 à 6, **caractérisée en ce que** n correspond à des valeurs comprises entre 2 et 50, de préférence comprises entre 2 et 20, et notamment comprises entre 3 et 10, les valeurs 4, 5, 6, 7 et 8 étant particulièrement préférées.

8. Utilisation de la combinaison selon l'une des revendications 1 à 7 en tant que précurseur de parfum.

9. Utilisation selon la revendication 8, **caractérisée en ce que** l'on met en oeuvre ladite combinaison en association avec d'autres parfums.

10. Agents de lavage ou de nettoyage, assouplissants ou produits cosmétiques, contenant une combinaison entre
(a) un composé de type aminoalcool répondant à la formule générale (I) 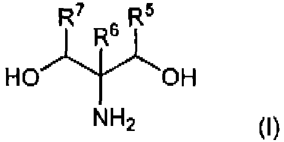 dans laquelle R⁵, R⁶, R⁷ représentent, indépendamment l'un de l'autre, H ou un résidu hydrocarboné lequel peut être acyclique ou cyclique, substitué ou non-substitué, ramifié ou linéaire ainsi que saturé ou insaturé, ou des mélanges de ces composés,
(b) un aldéhyde odorant et/ou une cétone odorante, et
(c) un ester d'acide silicique répondant à la formule générale (II) : 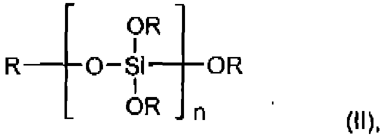 dans laquelle tous les R sont choisis, indépendamment l'un de l'autre, dans le groupe contenant H, les résidus hydrocarbonés en C₁₋₆ linéaires ou ramifiés, saturés ou insaturés, substitués ou non-substitués, et les radicaux d'alcools odorants, et n correspond à des valeurs comprises entre 2 et 100, et
au moins 10 % en mol, de préférence au moins 20 % en mol, et avec une préférence particulière même plus de 40 % en mol, des résidus R de la formule générale (II) étant choisis dans le groupe constitué des résidus des alcools odorants 10-undécén-1-ol, 2,6-diméthylheptan-2-ol, 2-méthylbutanol, 2-méthylpentanol, 2-phénoxy-éthanol, 2-phénylpropanol, 2-tert-butyl-cyclohexanol, 3,5,5-triméthyl-cyclohexanol, 3-hexanol, 3-méthyl-5-phenylpentanol, 3-octanol, 3-phénylpropanol, 4-hepténol, 4-isopropylcyclohexanol, 4-tert-butylcyclohexanol, 6,8-diméthyl-2-nonanol, 6-nonén-1-ol, 9-décén-1-ol, alcool alpha-méthylbenzylique, alpha-terpinéol, salicylate d'amyle, alcool benzylique, salicylate de benzyle, bêta-terpinéol, salicylate de butyle, citronellol, salicylate de cyclohexyle, décanol, dihydro-myrcénol, diméthylbenzylcarbinol, diméthylheptanol, diméthyloctanol, salicylate d'éthyle, éthylvanilline, eugénol, farnésol, géraniol, heptanol, salicylate d'hexyle, isobornéol, isoeugénol, isopulégol, linalool, menthol, myrténol, n-hexanol, nérol, nonanol, octanol, p-menthane-7-ol, phényléthanol, phénol, salicylate de phényle, tétrahydrogéraniol, tétrahydrolinalool, thymol, trans-2-cis-6-nonadiénol, trans-2-nonén-1-ol, trans-2-octénol, undécanol, vanilline, alcool cinnamique et de leurs mélanges.

11. Procédé de prolongement de la perception des parfums dégagés par des agents de lavage ou de nettoyage, des assouplissants ou des produits cosmétiques ou par les surfaces dures traitées avec ceux-ci, **caractérisé en ce que** l'on ajoute la combinaison selon l'une des revendications 1 à 7 auxdits agents de lavage ou de nettoyage, assouplissants ou produits cosmétiques, le parfum étant ensuite libéré par hydrolyse.

12. Procédé de réduction de mauvaises odeurs par mise en oeuvre d'une combinaison entre
(a) un composé de type aminoalcool répondant à la formule générale (I) 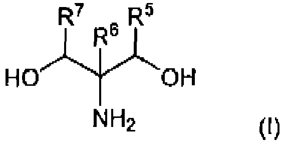 dans laquelle R⁵, R⁶, R⁷ représentent, indépendamment l'un de l'autre, H ou un résidu hydrocarboné lequel peut être acyclique ou cyclique, substitué ou non-substitué, ramifié ou linéaire ainsi que saturé ou insaturé, ou des mélanges de ces composés,
(b) un aldéhyde odorant et/ou une cétone odorante
(c) un ester d'acide silicique répondant à la formule générale (II) : 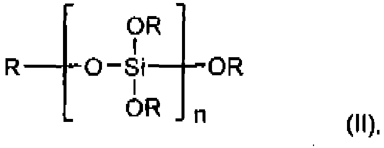 dans laquelle tous les R sont choisis, indépendamment l'un de l'autre, dans le groupe contenant H, les résidus hydrocarbonés en C₁₋₆ linéaires ou ramifiés, saturés ou insaturés, substitués ou non-substitués, et les radicaux d'alcools odorants, et n correspond à des valeurs comprises entre 2 et 100, et
au moins 10 % en mol, de préférence au moins 20 % en mol, et avec une préférence particulière même plus de 40 % en mol, des résidus R de la formule générale (II) étant choisis dans le groupe constitué des résidus des alcools odorants 10-undécén-1-ol) 2,6-diméthyl-heptan-2-ol, 2-méthylbutanol, 2-méthylpentanol, 2-phénoxyéthanol, 2-phénylpropanol, 2-tert-butyl-cyclohexanol, 3,5,5-triméthylcyclo-hexanol, 3-hexanol, 3-méthyl-5-phenylpentanol, 3-octanol, 3-phényl-propanol, 4-hepténol, 4-isopropylcyclohexanol, 4-tert-butyl-cyclohexanol, 6,8-diméthyl-2-nonanol, 6-nonén-1-ol, 9-décén-1-ol, alcool alpha-méthylbenzylique, alpha-terpinéol, salicylate d'amyle, alcool benzylique, salicylate de benzyle, bêta-terpinéol, salicylate de butyle, citronellol, salicylate de cyclohexyle, décanol, dihydro-myrcénol, diméthylbenzylcarbinol, diméthylheptanol, diméthyloctanol, salicylate d'éthyle, éthylvanilline, eugénol, farnésol, géraniol, heptanol, salicylate d'hexyle, isobornéol, isoeugénol, isopulégol, linalool, menthol, myrténol, n-hexanol, nérol, nonanol, octanol, p-menthane-7-ol, phényléthanol, phénol, salicylate de phényle, tétrahydrogéraniol, tétrahydrolinalool, thymol, trans-2-cis-6-nonadiénol, trans-2-nonén-1-ol, trans-2-octénol, undécanol, vanilline, alcool cinnamique et de leurs mélanges.
